# EUROPEAN PATENT APPLICATION

(11) **EP 4 193 961 A1**
(43) Date of publication of application: **14.06.2023**
(21) Application number: 21852782.8
(22) Date of filing: 04.06.2021
(51) Int. Cl.: A61D 9/00, A61F 5/01, A61F 5/058, A61F 13/06, A01K 13/00

(54) **KNEECAP BRACE**

(30) Priority: 06.08.2020 KR 20200098632; 14.10.2020 KR 20200132893
(71) Applicant: Wholesumbrands Co., Ltd., Seoul 06192 (KR)
(72) Inventor: SEO, Sang Woo, Seoul 03025 (KR); RO, Min Kyu, Seoul 03631 (KR); KIM, Hyun Dong, Seoul 03631 (KR)
(74) Representative: AWA Sweden AB
(86) International application number: PCT/KR2021/007040
(87) International publication number: WO 2022/030742

(57) **Abstract**

A kneecap brace, according to an embodiment of the present invention, comprises: a protective guard comprising a protective guard main body which covers the area surrounding a kneecap of a pet animal, a kneecap-accommodating hole, which is formed in a location corresponding to the kneecap, and trackers, which hold the kneecap from either side of the kneecap-accommodating hole; and main straps which fix the location at which the protective guard is worn.

## Description

### TECHNICAL FIELD

Embodiments of the disclosure relate to kneecap braces, and more particularly to kneecap braces for pets.

### BACKGROUND

Kneecap (patella) dislocations have been introduced among various disorders in pets.

Kneecap dislocations refer to a disorder that occurs when the kneecap located in the knee joint of a hind limb is dislocated inwards/outwards.

Mainly, kneecap dislocations occur more frequently in small dogs, which are easy to raise even indoors and thus are loved by people.

Kneecap dislocations can occur as a congenital disorder or as an acquired disorder caused by external shocks. For example, in the case of small dogs, the femoral trochlear groove is congenitally shallow and can thus easily slip out of place, in which case the problem of dislocating the kneecap inwards the body may occur.

If the kneecap is dislocated, ligaments and muscles attached to the kneecap can grow abnormally, which may affect the walking posture.

Therefore, many people who raise pet dogs have a lot of interest in preventing and taking care of kneecap dislocations.

As a prior art document related to the present disclosure, there is Korean Patent Application Publication No. 10-2019-0127446 (13 November, 2019: date of publication), in which document a brace preventing kneecap dislocation is disclosed.

### SUMMARY

It is an object of the present disclosure to provide a kneecap brace having an angle limiting function so that the angle of a leg does not deviate from a normal range.

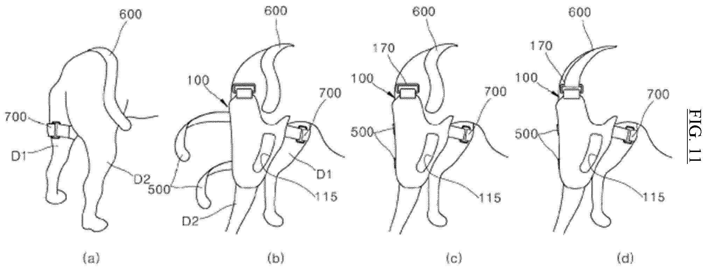

It is another object of the present disclosure to provide a kneecap brace capable of holding both sides of a kneecap in the form of figure 11 without compressing ligaments connected above and below the kneecap when worn.

It is yet another object of the present disclosure to provide a kneecap brace that is excellent in flexibility and cushioning feel and, accordingly, is easy to wear and comfortable to wear.

The objects of the present disclosure are not limited to the objects mentioned above, and other objects and advantages of the present disclosure that have not been mentioned can be understood by the following description, and will be understood more clearly by embodiments of the present disclosure. Furthermore, it will be readily appreciated that the objects and advantages of the present disclosure can be realized by the means indicated in the claims and combinations thereof.

According to an embodiment of the present disclosure for achieving the one object described above, there may be provided a kneecap brace having an angle limiting function so that the angle of a leg does not deviate from a normal range. In addition, according to an embodiment of the present disclosure, there may be provided a kneecap brace capable of holding both sides of a kneecap in the form of figure 11 without compressing ligaments connected above and below the kneecap when worn. Further, according to an embodiment of the present disclosure, there may be provided a kneecap brace that is excellent in flexibility and cushioning feel and, accordingly, is easy to wear and comfortable to wear.

A kneecap brace in accordance with one embodiment of the present disclosure, comprises: a protective guard comprising a protective guard main body configured to wrap around surrounding areas of a kneecap of a pet, a kneecap-accommodating hole formed at a position corresponding to the kneecap on an inner side of the protective guard main body, and a tracker configured to hold the kneecap on both sides of the kneecap-accommodating hole on the basis of the kneecap-accommodating hole; and a main strap connected by a set length on at least one side of the protective guard and configured to fix a wearing position of the protective guard.

The protective guard main body comprises: a first main body configured to wrap around one side of the surrounding areas of the kneecap; and a second main body configured to wrap around the other side of the surrounding areas of the kneecap on the basis of the kneecap-accommodating hole, and symmetrically connected to the first main body via remaining portions other than the kneecap-accommodating hole, the first main body is formed to wrap around an outer leg portion of the kneecap when the protective guard is worn, the second main body is formed to wrap around an inner leg portion of the surrounding areas of the kneecap when the protective guard is worn, and the first main body may have a larger area than the area of the second main body.

The protective guard main body comprises: a fabric part made of a first material with flexibility, and having a cushioning feeling by having a predetermined air layer formed therein; and a liner part stacked on an inner side of the fabric part and made of a second material having low friction with a skin of the pet when the protective guard is worn, and the first material may be made of a neoprene material, and the second material may be made of a polyamide-based synthetic resin material. As the first material is made of the neoprene material, there are advantages that it has good flexibility and can be worn regardless of size, and due to the nature of the materials, it has strength and elasticity and thus has the advantage of being able to compress after putting it on. Further, there are advantages of being able to prevent the occurrence of lifting and looseness between the protective guard main body and the wearing portion of the surrounding areas of the kneecap, and to prevent the protective guard main body from shaking or loosening after being put on. Moreover, as a predetermined air layer is formed inside the fabric part, there is an advantage of being able to improve a cushioning feeling and to provide comfort when worn. A polyamide-based synthetic resin is used for the second material, thereby having an advantage of being harmless to the body of the pet even when worn for an extended time. Polyamide-based synthetic resins are medical-grade fabric materials and can be placed at a position where they can come into contact with the skin of the pet, and has an advantage of having the lowest coefficient of friction (COF) among medical-grade fabric materials and being soft. Therefore, there is an advantage of reducing friction with the skin of the pet.

The protective guard main body further comprises: an edge part finished along an outer edge of the protective guard main body and made of a third material. A soft synthetic covering material may be used as the third material. The edge part can minimize friction with the skin of the pet when worn for a prolonged time and can increase durability by finishing the edge of the main body.

The tracker may have a shape of figure 11 that is symmetrical left and right on the basis of the kneecap-accommodating hole so as to wrap around ligaments in the surrounding areas of the kneecap on both sides of the kneecap-accommodating hole when the protective guard is worn. The kneecap is connected to the upper and lower ligaments. The tracker can hold both sides of the kneecap in the form of figure 11 without compressing the upper and lower ligaments connected to the kneecap. If the position of the kneecap is not taken into account or in the case of a form that simply wraps around the kneecap in an annular or donut form, compression may be applied above and below the kneecap, which can cause damage to the ligaments above and below the kneecap, and it is highly likely to develop into osteoarthritis or cruciate ligament rupture. The tracker must have the shape of figure 11 and may have a symmetrical shape on both left and right sides with respect to the kneecap-accommodating hole.

The kneecap-accommodating hole has a round shape that is formed longer in vertical length than horizontal length, the tracker comprises: a first tracker body arranged long vertically along a boundary on one side of the kneecap-accommodating hole; and a second tracker body arranged long vertically along a boundary on the other side of the kneecap-accommodating hole so as to have a shape that is symmetrical left and right with the first tracker body on the basis of a center line of the kneecap-accommodating hole, the length of each of the first and second tracker bodies may be formed to be longer than the vertical length of the kneecap-accommodating hole, and the first and second tracker bodies may be made of a silicon material.

The tracker comprises: a straight part formed to be straight along both sides of the kneecap-accommodating hole; and an oblique extension part connected to an upper end of the straight part and having a shape diverging in a left-right direction at a first set angle from an upper side of the kneecap-accommodating hole.

The inner part of the tracker may have a concave curved shape so as to be puckered toward the surrounding areas of the kneecap when the protective guard is worn, and the outer part of the tracker may have a curved shape with a convex outer surface.

The straight part holds both sides of the kneecap in the form of figure 11, and has a shape with an open lower end so as not to compress the ligaments located on the lower side of the kneecap. In this way, damage to the ligaments located below the kneecap can be prevented.

The oblique extension part has a shape with an open upper end to protect the ligaments located on the upper side of the kneecap, and can prevent damage to the ligaments located above the kneecap by having a shape that diverges left and right from the upper end of the straight part on the basis of the kneecap-accommodating hole.

In addition, the kneecap brace in accordance with one embodiment of the present disclosure further comprises: support members detachably coupled to an inner side of each of the first and second main bodies and configured to reinforce a supporting force according to conditions of the kneecap of the pet. The support members include integrated support members and rotational support members, each of which may be replaced and used for each step. For example, by dividing into a step of wearing the protective guard without the support members (step 1), a step of wearing the protective guard into which the rotational support members have been inserted (step 2), and a step of wearing the protective guard into which the integrated support members have been inserted (step 3), it is possible to improve the health of the kneecap joint of the pet by adjusting for each step according to the condition of the pet. In other words, steps 2 and 3 provide the effect of reinforcing the supporting force when the kneecap condition of the pet is poor and lacks strength.

The support members are lightweight metallic materials and may use 60 and 50 series aluminum, which has required strength and rigidity.

The support members comprise: first support bodies located on upper sides of the first and second main bodies; and second support bodies connected to lower ends of the first support bodies and located on lower sides of the first and second main bodies, and the first support bodies and the second support bodies may be connected to have a set inclination angle (integrated support members).

In addition, the kneecap brace in accordance with one embodiment of the present disclosure may be provided in a structure in which the first and second support bodies are not configured in an integrated structure, but each of the first and second support bodies is provided in a separate type and is connected by at least one hinge part (rotational support members). There may be further provided a rotation angle limiting body to limit the variability of an excessive inclination angle by the hinge part. The rotation angle limiting body may be located at the upper end of the second support body, and can physically limit the inclination angle from getting larger or smaller due to the first support body deviating from the set range around the hinge part.

The first and second support bodies may be adjusted in the inclination angle between the first and second support bodies within a set range. The hinge part may be located between the tibia and the femur. As the hinge part is located between the tibia, i.e., the bone below the knee, and the femur, i.e., the bone above the knee, symmetry can be made on the basis of the knee when worn, and thus a supporting force can be exhibited more precisely with the three-point pressure principle.

The main strap comprises: a first main strap configured to be spaced upward from the kneecap when the protective guard is worn and to wrap around an upper middle part of a thigh of the pet; and a second main strap located at a lower end of the first main strap, and configured to be spaced downward from the kneecap and to wrap around an upper part of a shin of the pet. The main strap is fastened to the upper and lower parts of the protective guard main body, and may be provided with Velcro for attachment and detachment at each distal end. The main strap can improve discomfort when the joint is folded by reducing the space behind and next to the knee while completely wrapping around the upper part of the ankle joint (hock).

In addition, the kneecap brace in accordance with one embodiment of the present disclosure may further include a waist strap and an auxiliary belt.

The waist strap may be fastened to a fastening ring located in the outer periphery of the protective guard main body. The waist strap can prevent the protective guard main body from hanging down after the protective guard is put on. The waist strap may be provided in the form of an adjustable length.

The auxiliary belt may be connected to the waist strap. The auxiliary belt may be fastened to the leg opposite to the leg on which the protective guard is worn. The auxiliary belt, together with the waist strap, prevents the protective guard from hanging down and is worn on the healthy opposite leg, and prevents the waist strap from touching the genitals of the pet, thereby improving the wearing comfort without irritating the groin region of the pet.

Further, the kneecap brace in accordance with one embodiment of the present disclosure may further include a pad (not shown). The pad can be used by inserting it into the inside of the protective guard main body, and may be made of a silicon material or the like, and can protect the skin of a pet that is sensitive to skin irritation and further improve the wearing comfort.

According to the embodiments of the present disclosure, there is an advantage of having an angle limiting function so that the angle of a leg does not deviate from a normal range. In addition, there is an advantage of being capable of holding both sides of a kneecap in the form of figure 11 without compressing ligaments connected above and below the kneecap when worn. Moreover, there is an advantage of being excellent in flexibility and cushioning feel and, accordingly, being easy to wear and comfortable to wear.

In addition to the effects described above, specific effects of the present disclosure will be described together while describing specific details for carrying out the invention below.

### BRIEF DESCRIPTION OF THE DRAWINGS

FIG. 1 is a view of a kneecap brace in accordance with one embodiment of the present disclosure viewed in the perspective direction;
FIG. 2 is a view of a kneecap brace in accordance with one embodiment of the present disclosure viewed from the front direction;
FIG. 3 is a view of a kneecap brace in accordance with one embodiment of the present disclosure viewed from the rear direction;
FIG. 4 is a view of a kneecap brace in accordance with one embodiment of the present disclosure viewed from the side direction;
FIG. 5 is a view of a kneecap brace in accordance with one embodiment of the present disclosure viewed from the plan direction;
FIG. 6 is a view showing a state in which a kneecap brace in accordance with one embodiment of the present disclosure is folded and stored;
FIG. 7 is a view enlarging and showing the placement position and shape of a tracker applied to a kneecap brace in accordance with one embodiment of the present disclosure;
FIGS. 8 and 9 are views showing a support device applied to a kneecap brace in accordance with one embodiment of the present disclosure in a state of having been pulled out;
FIG. 10 is a view showing an exemplary shape of the support device shown in FIGS. 8 and 9;
FIG. 11 is a view showing an order of wearing a kneecap brace in accordance with one embodiment of the present disclosure; and
FIG. 12 is a photograph showing a state in which a kneecap brace in accordance with one embodiment of the present disclosure is worn on a pet.

### DETAILED DESCRIPTION OF THE PREFERRED EMBODIMENTS

Hereinafter, with reference to the drawings, an embodiment of the present disclosure will be described in detail so that a person skilled in the art can easily carry it out. The present disclosuremay be embodied in many different forms and is not limited to the embodiments set forth herein.

In order to clearly describe the present disclosure, parts irrelevant to the description are omitted, and the same reference numerals are given to the same or similar components throughout the specification. Some embodiments of the present disclosure are also described in detail with reference to exemplary figures. In adding reference numerals to components of each drawing, the same components may have the same numerals as much as possible even if they are displayed on different drawings. In addition, in describing the present disclosure, if it is determined that a detailed description of a related known configuration or function may obscure the gist of the present disclosure, the detailed description may be omitted.

In describing the components of the present disclosure, when a component is described as "connected", "coupled", or "connected" to another component, the component may be directly connected or connected to the other component. However, it will be understood that other components may be "interposed" between each component, or each component may be "connected", "coupled", or "connected" through other components.

As is well known, kneecap (patella) dislocation is one of the various disorders in pets, and refers to a disorder that occurs when the kneecap located in the knee joint of a hind limb is dislocated inwards/outwards. Mainly, kneecap dislocations occur frequently in small dogs, which are easy to raise even indoors and thus are loved by people.

Kneecap dislocations can occur as a congenital disorder or as an acquired disorder caused by external shocks. If the kneecap is dislocated, ligaments and muscles attached to the kneecap can grow abnormally, which may affect the walking posture.

According to embodiments of the present disclosure, there is provided a kneecap brace that has an angle limiting function so that the angle of a leg does not deviate from a normal range, that is capable of holding both sides of a kneecap in the form of figure 11 without compressing ligaments connected above and below the kneecap when worn, and that is excellent in flexibility and cushioning feel and, accordingly, is easy to wear and comfortable to wear.

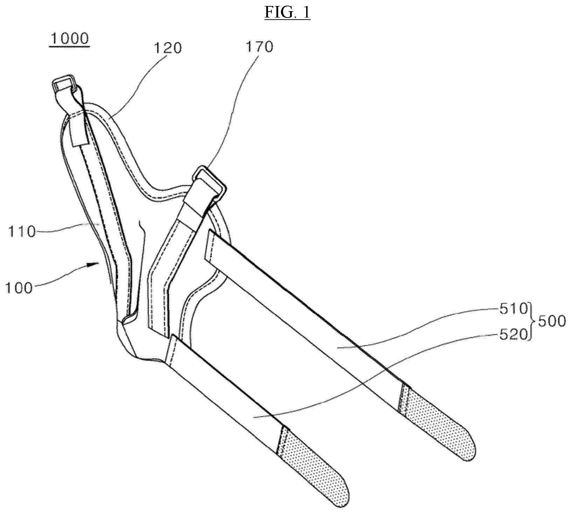

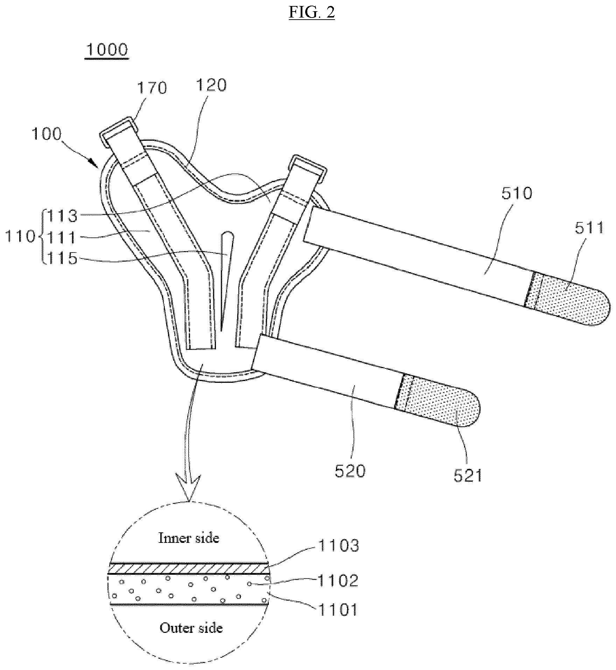

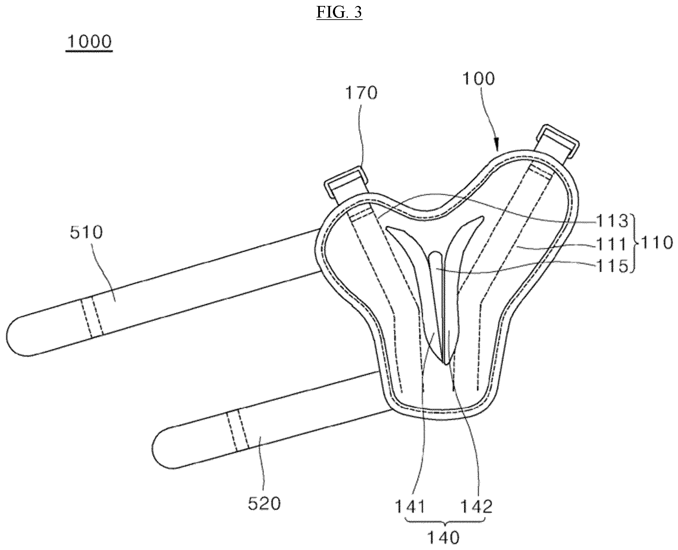

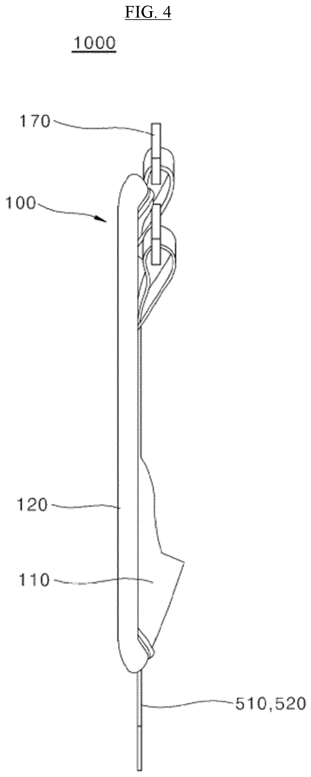

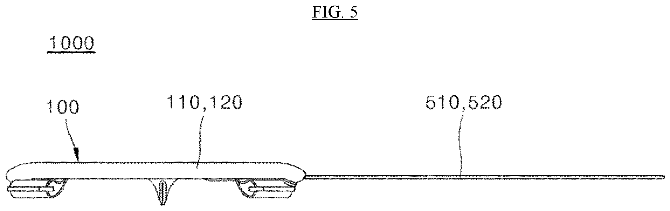

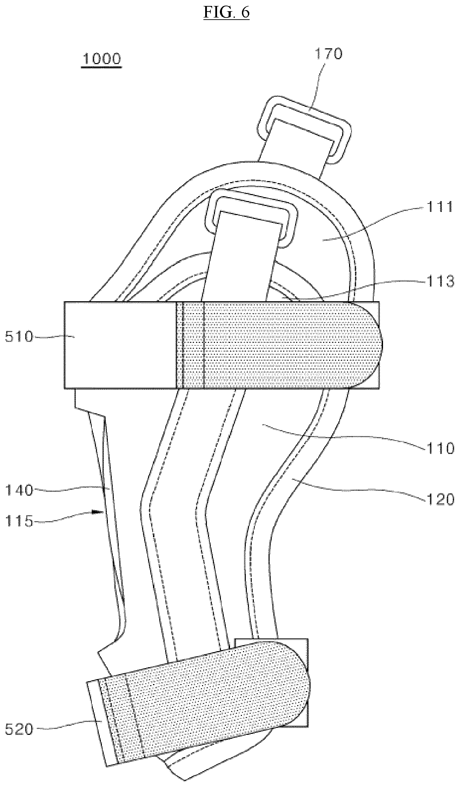

In the drawings, FIG. 1 is a kneecap brace viewed in the perspective direction, FIG. 2 is the kneecap brace viewed from the front direction, FIG. 3 is the kneecap brace viewed from the rear direction, FIG. 4 is the kneecap brace viewed from the side direction, FIG. 5 is the kneecap brace viewed from the plan direction, and FIG. 6 shows a state in which the kneecap brace is folded and stored.

As shown, the kneecap brace 100 in accordance with one embodiment of the present disclosure includes a protective guard 100 and a main strap 500.

The protective guard 100 includes a protective guard main body 110, a kneecap-accommodating hole 115, and a tracker 140.

The protective guard main body 1100 is a member that wraps around the surrounding areas of a kneecap of a pet.

The kneecap-accommodating hole 115 refers to a hole formed at a position corresponding to the kneecap on the inner side of the protective guard main body 110.

The tracker 140 is a member that holds the kneecap on both sides of the kneecap-accommodating hole 115 on the basis of the kneecap-accommodating hole 115.

The main strap 500 is connected by a set length on at least one side of the protective guard 100 and fixes the wearing position of the protective guard 100.

Described specifically, the protective guard main body 110 includes a first main body 111 and a second main body 113.

The first main body 111 is formed to wrap around one side of the surrounding areas of the kneecap.

The second main body 113 is formed to wrap around the other side of the surrounding areas of the kneecap on the basis of the kneecap-accommodating hole. In addition, the second main body 113 may be integrally connected to the first main body 111 in a symmetrical manner with the first main body 111 via the remaining portions other than the kneecap-accommodating hole 115.

Described more specifically, the first main body 111 may be formed to wrap around the outer leg portion of the kneecap when the protective guard 100 is worn.

In contrast, the second main body 113 may be formed to wrap around the inner leg portion of the surrounding areas of the kneecap when the protective guard 100 is worn.

The first main body 111 may have a larger area and a shape that protrudes upward more than the second main body 113. According to this shape, it is possible to improve the fit of wearing by taking into account the body structure of the outer and inner sides of a leg of a pet, and provide comfort even when worn for a prolonged time by reducing unnecessary interference with the body of the pet.

The protective guard main body 110 includes a fabric part 1101 and a liner part 1103 (see FIG. 2).

The fabric part 1101 is made of a first material having flexibility, and a predetermined air layer 1102 may be formed therein to have a cushioning feeling.

The liner part 1103 is stacked on the inner side of the fabric part 1101, and may be made of a second material with low friction even if it comes into contact with the skin of the pet when the protective guard 100 is worn.

For example, the first material may be made of a neoprene material. The second material may be made of a polyamide-based synthetic resin material.

As the first material is made of the neoprene material, there are advantages that it has good flexibility and can be worn regardless of size. In addition, due to the nature of the materials, it has strength and elasticity and thus has the advantage of being able to compress after putting it on. Further, it is possible to prevent the occurrence of lifting and looseness between the protective guard main body and the wearing portion of the surrounding areas of the kneecap, and to prevent the protective guard main body 110 from shaking or loosening after being put on. Moreover, if a predetermined air layer is formed inside the fabric part 1101, it is possible to improve a cushioning feeling and thus provide comfort when worn, and the neoprene material contains an air layer and thus is suitable for use as a material for the fabric part 1101.

A polyamide-based synthetic resin is used for the second material, unlike the first material, thereby having an advantage of being harmless to the body of the pet even when worn for an extended time.

Polyamide-based synthetic resins are medical-grade fabric materials, and are advantageous for use as the liner part 1103 placed at a position where it can come into contact with the skin of the pet. In addition, polyamide-based synthetic resins have the lowest coefficient of friction (COF) among medical-grade fabric materials and have a soft property, and thus can reduce friction with the skin of the pet.

The protective guard main body 110 further includes an edge part 120 finished along the outer edge of the protective guard main body and made of a third material.

A soft synthetic covering material may be used as the third material. The edge part 120 is a portion that can come into contact with the skin of the pet, and can minimize friction with the skin of the pet when worn for a prolonged time by using a soft synthetic covering material, and can prevent damage and wear and increase durability by finishing the edge of the protective guard main body 110.

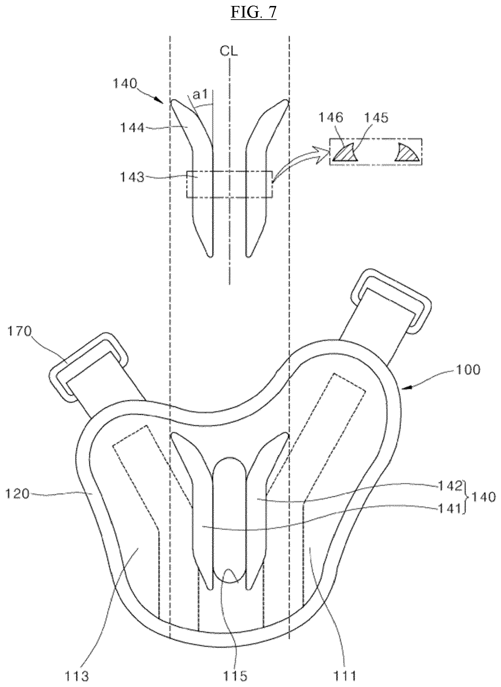

FIG. 7 is a view enlarging and showing the placement position and shape of a tracker applied to a kneecap brace in accordance with one embodiment of the present disclosure.

As shown, the tracker 140 may have a shape of figure 11 that is symmetrical left and right on the basis of the kneecap-accommodating hole 115 so as to wrap around ligaments in the surrounding areas of the kneecap on both sides of the kneecap-accommodating hole 115 when the protective guard 110 is worn.

As is well known, the kneecap is connected to the upper and lower ligaments. The tracker 114 in the shape of figure 11 can stably hold both sides of the kneecap in the form of figure 11 without compressing the upper and lower ligaments connected to the kneecap.

If the position of the kneecap is not taken into account or in the case of a form that simply wraps around the kneecap in an annular or donut form (some conventionally introduced forms), compression may be applied above and below the kneecap, which can cause damage to the ligaments above and below the kneecap, and it is highly likely to develop into osteoarthritis or cruciate ligament rupture.

Accordingly, the tracker 140 must have the shape of figure 11 and preferably has a symmetrical shape on both left and right sides on the basis of the kneecap-accommodating hole 115.

The kneecap-accommodating hole 115 has a round shape that is formed longer in the vertical length than the horizontal length.

For example, the tracker 140 may comprise a first tracker body 141 and a second tracker body 142.

The first tracker body 141 may be arranged long vertically along the boundary on one side of the kneecap-accommodating hole 115.

The second tracker body 141 may be arranged long vertically along the boundary on the other side of the kneecap-accommodating hole 115 so as to have a shape that is symmetrical left and right with the first tracker body 141 on the basis of the center line (C.L, see FIG. 7) of the kneecap-accommodating hole 115.

In this case, the length of each of the first and second tracker bodies 141 and 142 may be formed longer than the vertical length of the kneecap-accommodating hole 115, and the first and second tracker bodies 141 and 142 may be made of a silicon material. As being configured in this way, the tracker 140 in the shape of figure 11 including the first and second tracker bodies 141 and 142 can stably hold both sides of the kneecap and prevent damage to the ligaments above and below the kneecap.

In addition, the tracker 140 (141 and 142) includes a straight part 143 and an oblique extension part 144.

The straight part 143 may be formed to be straight along both sides of the kneecap-accommodating hole 115. The oblique extension part 144 may be connected to the upper end of the straight part 143 and may have a shape diverging in the left-right direction at a first set angle from the upper side of the kneecap-accommodating hole 115.

Further, an inner part 145 of the tracker 140 may have a concave curved shape so as to be puckered toward the surrounding areas of the kneecap when the protective guard 100 is worn. In contrast, an outer part 146 of the tracker 140 may have a curved shape with a convex outer surface.

The straight part 143 holds both sides of the kneecap in the form of figure 11, and has a shape with an open lower end so as not to compress the ligaments located on the lower side of the kneecap. In this way, damage to the ligaments located below the kneecap can be prevented.

The oblique extension part 144 has a shape with an open upper end to protect the ligaments located on the upper side of the kneecap, and can prevent damage to the ligaments located above the kneecap by having a shape that diverges left and right by a predetermined angle a1 from the upper end of the straight part 143 on the basis of the kneecap-accommodating hole 115.

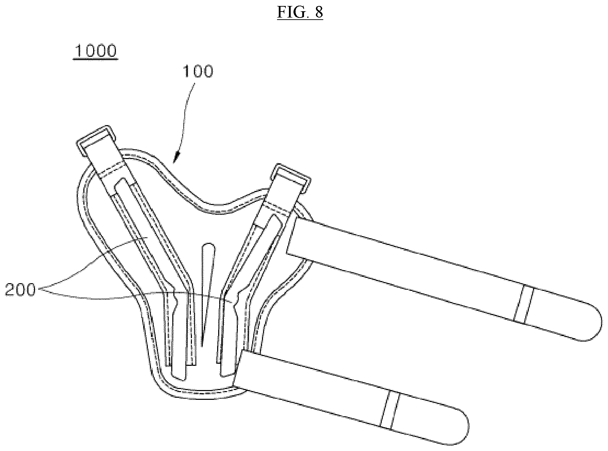

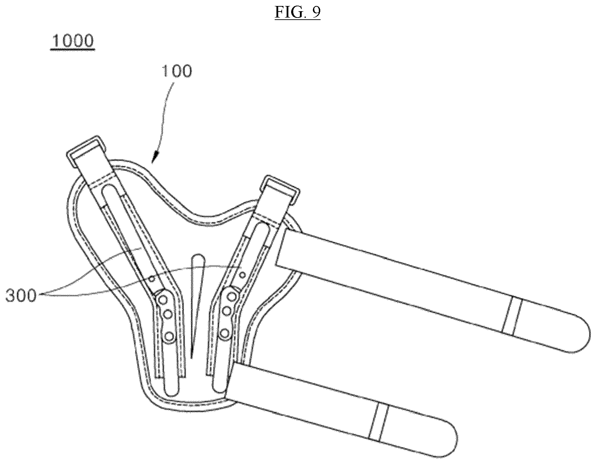

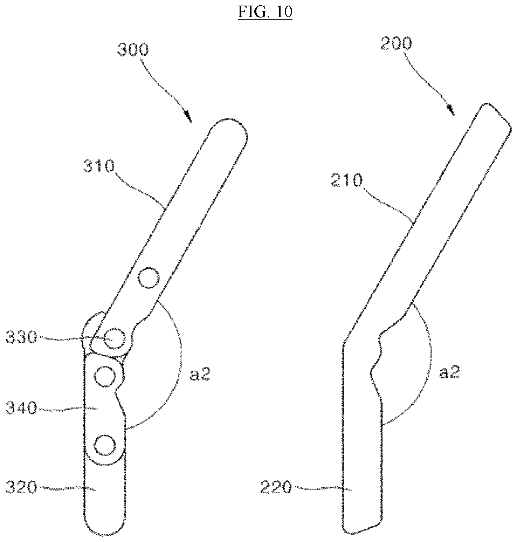

FIGS. 8 and 9 are views showing a support device applied to a kneecap brace in accordance with one embodiment of the present disclosure in a state of having been pulled out, and FIG. 10 is a view showing an exemplary shape of the support device shown in FIGS. 8 and 9.

As shown, the kneecap brace 1000 in accordance with one embodiment of the present disclosure further includes support members 200 and 300.

The support members 200 and 300 may be detachably coupled to the inner side of each of the first and second main bodies 111 and 113. The support members 200 and 300 may serve to reinforce the supporting force according to the condition of the kneecap of the pet.

The support members 200 and 300 include integrated support members 200 and rotational support members 300. Each of the integrated support members 200 and the rotational support members 300 may be replaced and used for each step.

For example, it can be divided into a step of wearing the protective guard 100 only without the support members (step 1), a step of wearing the protective guard 100 into which the rotational support members 300 have been inserted (step 2), and a step of wearing the protective guard 100 into which the integrated support members 200 have been inserted (step 3). In this way, it is possible to improve the health of the kneecap joint of the pet by adjusting for steps 1, 2, and 3 according to the condition of the pet. Steps 2 and 3 can reinforce the supporting force when the kneecap condition of the pet is poor and lacks strength.

The support members 200 and 300 are lightweight metallic materials and may use 60 and 50 series aluminum, which has required strength and rigidity. However, it is not necessarily limited to this, and it can be changed into and use various lightweight metallic materials that are obvious to those of ordinary skill in the art as well.

The integrated support member 200 includes a first support body 210 and a second support body 220 (see FIGS. 8 and 10).

The first support body 210 is located on the upper side of the first and second main bodies 111 and 113. The second support body 220 is connected to the lower end of the first support body 210 and is located on the lower side of the first and second main bodies 111 and 113.

The first support body 210 and the second support body 220 may be connected to have a set inclination angle a2. For example, it can have an inclination angle a2 of 150 degrees. The magnitude of the set inclination angle a2 may be adjusted appropriately to the tibofemoral angle within a range of 130 degrees to 160 degrees.

The rotational support member 300 includes a first support body 310 and a second support body 320 (see FIGS. 9 and 10).

The first support body 310 is located on the upper side of the first and second main bodies 111 and 113. The second support body 320 is connected to the lower end of the first support body 310 and is located on the lower side of the first and second main bodies 111 and 113.

Here, for the rotational support member 300, each of the first and second support bodies 310 and 320 may be provided in separate types, unlike the integrated support member 200 described above.

And the first and second support bodies 310 and 320 may be provided in a structure to be connected by at least one hinge part 330. Accordingly, the inclination angle a2 between the first support body 310 and the second support body 320 may be adjusted appropriately to the tibofemoral angle within a range of 130 degrees to 160 degrees. Preferably, it may be 150 degrees.

In addition, a rotation angle limiting body 340 may be further provided so as to limit the rotation range by the hinge part 330. The rotation angle limiting body 340 may be located at the upper end of the second support body 320. The rotation angle limiting body 340 limits the rotation range by physically preventing the first support body 310 from rotating around the hinge part 330 out of a set angle range.

As such, the first and second support bodies 310 and 320 may be adjusted in the inclination angle between the first and second support bodies 310 and 320 within a set angle range (e.g., 130 degrees to 160 degrees, or the like).

In this case, the hinge part 300 is preferably located between the tibia and the femur. As the hinge part 300 is located between the tibia, i.e., the bone below the knee, and the femur, i.e., the bone above the knee, symmetry can be made on the basis of the knee when the protective guard 100 is worn, and thus a supporting force can be provided more stably with the three-point pressure principle.

The main strap 500 includes a first main strap 510 and a second main strap 520.

The first main strap 510 is a member in the form of a string or band that is spaced upward from the kneecap when the protective guard 100 is worn and wraps around the upper middle part of the thigh of the pet. The second main strap 520 is located at the lower end of the first main strap 510, and is a member in the form of a string or band that is spaced downward from the kneecap and wraps around the upper part of the shin of the pet.

In this way, the main strap 500 is fastened to the upper and lower parts of the protective guard main body 110, and may be provided with Velcro 511 and 521 for attachment and detachment at each distal end. For example, the distal end of the first main strap 510 may be provided with a first Velcro 511 and the distal end of the second main strap 520 may be provided with a second Velcro 521. Having this configuration, the main strap 500 can improve discomfort when the joint is folded by reducing the space behind and next to the knee while completely wrapping around the upper part of the ankle joint (hock).

FIG. 11 is a view showing an order of wearing a kneecap brace in accordance with one embodiment of the present disclosure.

As shown, the kneecap brace 1000 in accordance with one embodiment of the present disclosure may further include a waist strap 600 and an auxiliary belt 700.

The waist strap 600 may be fastened to a fastening ring 170 located in the outer periphery of the protective guard main body 110. The waist strap 600 prevents the protective guard main body 110 from hanging down after the protective guard 100 is put on. The waist strap 600 may be provided in the form of an adjustable length.

The auxiliary belt 700 may be connected to the waist strap 600. The auxiliary belt 700 may be fastened to the leg D1 opposite to the leg D2 on which the protective guard 100 is worn.

The auxiliary belt 700, together with the waist strap 600, prevents the protective guard 100 from hanging down, and is worn on the healthy opposite leg D1. By using the auxiliary belt 700, the waist strap 600 can be prevented from touching the genitals of the pet, thereby improving the wearing comfort without irritating the groin region of the pet.

Meanwhile, the kneecap brace 1000 in accordance with one embodiment of the present disclosure may further include a pad (not shown). The pad can be used by inserting it into the inside of the protective guard main body, and may be made of a silicon material or the like, and can protect the skin of the pet that is sensitive to skin irritation and further improve the wearing comfort.

Referring to FIG. 11, the auxiliary belt 700 to which the waist strap 600 is connected is fastened to the opposite leg D1 (see FIG. 11 (a)). Subsequently, the center of the kneecap-accommodating hole 115 of the protective guard 100 is positioned to be aligned with the kneecap (see FIG. 11 (a)). Subsequently, the main strap 500 is fastened (see FIG. 11 (a)). Next, the waist strap 600 is fastened to the fastening ring 170, thereby completing the put-on.

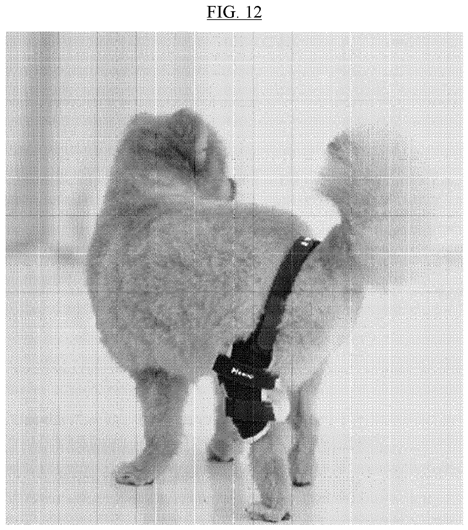

FIG. 12 is a photograph showing a state in which a kneecap brace in accordance with one embodiment of the present disclosure is worn on a pet. Referring to FIG. 12, a state in which the kneecap brace is worn on a leg of a pet dog can be confirmed.

As described above, the present disclosure has been described with reference to the drawings illustrated, but the present disclosure is not limited by the embodiments and drawings disclosed herein, and various modifications are made by those skilled in the art within the scope of the technical idea of the present disclosure. It is self-evident that this can be done. In addition, although the technical effects according to the configuration of the present disclosure have not been explicitly described and described while describing the embodiments of the present disclosure above, it is natural that the effects predictable by the corresponding configuration should also be recognized.

## Claims

1. A kneecap brace comprising:
a protective guard comprising:
a protective guard main body configured to wrap around surrounding areas of a kneecap of a pet;
a kneecap-accommodating hole formed at a position corresponding to the kneecap on an inner side of the protective guard main body; and
a tracker configured to hold the kneecap on both sides of the kneecap-accommodating hole on the basis of the kneecap-accommodating hole; and
a main strap connected by a set length on at least one side of the protective guard and configured to fix a wearing position of the protective guard.

2. The kneecap brace of claim 1, wherein the protective guard main body comprises:
a first main body configured to wrap around one side among the surrounding areas of the kneecap; and
a second main body configured to wrap around the other side of the surrounding areas of the kneecap on the basis of the kneecap-accommodating hole, and symmetrically connected to the first main body via remaining portions other than the kneecap-accommodating hole, and
wherein the first main body is formed to wrap around an outer leg portion of the kneecap when the protective guard is worn, and the second main body is formed to wrap around an inner leg portion of the surrounding areas of the kneecap when the protective guard is worn,
**characterized in that** the first main body has a larger area than the area of the second main body.

3. The kneecap brace of claim 2, wherein the protective guard main body comprises:
a fabric part made of a first material with flexibility, and having a cushioning feeling by having a predetermined air layer formed therein; and
a liner part stacked on an inner side of the fabric part and made of a second material having low friction with a skin of the pet when the protective guard is worn,
**characterized in that** the first material is made of a neoprene material, and the second material is made of a polyamide-based synthetic resin material.

4. The kneecap brace of claim 3, wherein the protective guard main body further comprises:
an edge part finished along an outer edge of the protective guard main body and made of a third material.

5. The kneecap brace of claim 3, **characterized in that** the tracker has a shape of figure 11 that is symmetrical left and right on the basis of the kneecap-accommodating hole so as to wrap around ligaments in the surrounding areas of the kneecap on both sides of the kneecap-accommodating hole when the protective guard is worn.

6. The kneecap brace of claim 5, wherein the kneecap-accommodating hole has a round shape that is formed longer in vertical length than horizontal length, and
the tracker comprises:
a first tracker body arranged long vertically along a boundary on one side of the kneecap-accommodating hole; and
a second tracker body arranged long vertically along a boundary on the other side of the kneecap-accommodating hole so as to have a shape that is symmetrical left and right with the first tracker body on the basis of a center line of the kneecap-accommodating hole,
**characterized in that** the first and second tracker bodies are made of a silicon material.

7. The kneecap brace of claim 6, wherein the tracker comprises:
a straight part formed to be straight along both sides of the kneecap-accommodating hole; and
an oblique extension part connected to an upper end of the straight part and having a shape diverging in a left-right direction at a first set angle from an upper side of the kneecap-accommodating hole.

8. The kneecap brace of claim 2, further comprising:
support members detachably coupled to an inner side of each of the first and second main bodies and configured to reinforce a supporting force according to conditions of the kneecap of the pet,
wherein the support members comprise:
first support bodies located on upper sides of the first and second main bodies; and
second support bodies connected to lower ends of the first support bodies and located on lower sides of the first and second main bodies,
**characterized in that** the first support bodies and the second support bodies are connected to have a set inclination angle.

9. The kneecap brace of claim 8, **characterized in that** each of the first and second support bodies is provided in a separate type and is connected by at least one hinge part, and
the first and second support bodies are adjusted in an inclination angle between the first and second support bodies within a set range.

10. The kneecap brace of claim 1, wherein the main strap comprises:
a first main strap configured to be spaced upward from the kneecap when the protective guard is worn and to wrap around an upper middle part of a thigh of the pet; and
a second main strap located at a lower end of the first main strap, and configured to be spaced downward from the kneecap and to wrap around an upper part of a shin of the pet.
